# DEMANDE DE BREVET EUROPEEN

(11) **EP 0 691 336 A1**
(43) Date de publication de la demande: **10.01.1996**
(21) Numéro de dépôt: 95420156.2
(22) Date de dépôt: 13.06.1995
(51) Int. Cl.: C07D 307/89, C08K 5/15, C09D 17/00

(54) **Nouveaux agents dispersants dérivés d'anhydride et nouvelles compositions polymériques chargées ainsi que leurs applications**

(30) Priorité: 08.07.1994 FR 9408782
(71) Demandeur: COATEX S.A., F-69730 Genay (FR)
(72) Inventeur: Blanchard, Pierre, F-01600 Reyrieux (FR); Trouve, Patrick, F-92140 Clamart (FR)

(57) **Abrégé**

Nouveaux dérivés d'anhydride utilisés comme agent dispersant de charges minérales et/ou organiques dans des matières thermoplastiques ou thermodurcissables.

Compositions thermoplastiques ou thermodurcissables, chargées, fluides et homogènes les contenant et leurs applications dans la fabrication des matières plastiques.

Nouveaux dérivés de formule 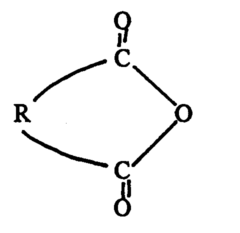
dans laquelle 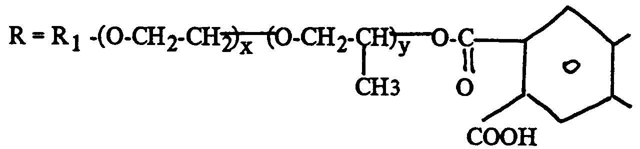
ou 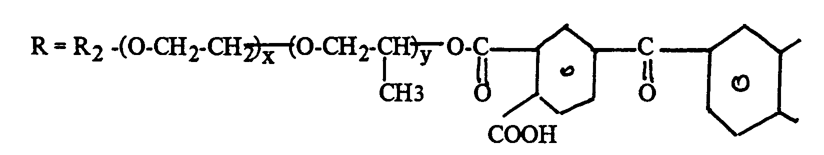
avec
- x et y des nombres allant de 0 à 100 inclus tels que la somme (x + y) varie de 0 à 100.
- R₁ un radical alkyle possédant jusqu'à 40 atomes de carbone si x ou y ne sont pas nuls et possédant de 10 à 40 atomes si x et y sont nuls ou bien un radical aryle, alkylaryle, arylalkyle, polyaryle ramifié ou non.
- R₂ un radical alkyle possédant jusqu'à 40 atomes de carbone quels que soient x et y ou bien un radical aryle, alkylaryle, arylalkyle, polyaryle ramifié ou non.

## Description

La présente invention concerne les dérivés d'anhydride utilisés comme agent dispersant de charges minérales et/ou organiques dans les matières thermoplastiques et thermodurcissables en vue d'obtenir des compositions polymériques hautement chargées, fluides et homogènes.

Elle concerne également ces dérivés utilisés comme agent dispersant de charges minérales et/ou organiques dans les résines destinées à être transformées aussi bien à froid qu'à chaud.

Cette invention se rapporte aussi aux compositions polymériques thermoplastiques ou thermodurcissables ou bien encore transformables à basse température comme à chaud, hautement chargées, fluides et homogènes contenant ledit agent dispersant.

Enfin cette invention est relative à l'utilisation de ces compositions polymériques chargées dans la fabrication des matières plastiques.

Pour diminuer le prix de revient des produits obtenus aussi bien à partir des compositions thermoplastiques que des résines thermodurcissables ou bien encore transformables à basse température comme à chaud il s'avère de plus en plus nécessaire d'augmenter la quantité de charge minérale et/ou organique dans les résines tout en conservant ou améliorant leurs propriétés physico-chimiques telles que par exemple mécaniques, thermiques, diélectriques, ou bien encore leurs propriétés esthétiques, ainsi que de chercher à obtenir une viscosité du mélange qui soit la plus faible possible à haut et bas taux de cisaillement. En effet, le fait de ne plus avoir de seuil d'écoulement permet à la résine d'être travaillée facilement à tous les stades de la fabrication. Dans le but d'introduire des charges dans ces résines, l'utilisation d'agents dispersants du type organo-phosphoré est bien connue.

Ainsi, on peut citer les demandes de brevets JP 61-101527, JP 62-207337, JP 62-235353 qui proposent l'introduction d'organo-phosphorés dans des mélanges contenant au maximum 100 parts de charge pour 100 parts de résine. Pour des concentrations de charge minérale supérieures allant jusqu'à 65 % en poids, le brevet US 4,183,843 revendique l'utilisation d'esters polaires de l'acide phosphorique. A faible taux d'utilisation, ces produits diminuent la viscosité des mélanges de carbonate de calcium et/ou d'hydroxyde d'aluminium et/ou de dioxyde de titane et/ou de silice et/ou d'argile avec les résines de type polyester insaturé sous haut cisaillement, mais la viscosité sous très faible cisaillement reste très importante ; il y a alors un seuil d'écoulement qui rend la manipulation des formulations difficile.

L'homme du métier connaît aussi des agents dispersants organophosphorés tels que ceux décrits dans les brevets (FR 2 582 310 - FR 2 602 236) utilisés dans les résines thermofusibles chargées ou bien encore des agents dispersants à base d'esters phosphoriques tels que ceux décrits dans le brevet EP 0 417 490 utilisés dans les résines thermodurcissables ou bien encore des agents dispersants organo-phosphorés à groupe polyaryle (FR 2 671 555 et FR 2 671 556) pouvant éliminer cet effet malgré un taux de charge pouvant aller jusqu'à 75 % en poids de la composition.

Cependant de tels agents présentent l'inconvénient d'inhiber la réaction pendant la formulation utilisant des dérivés organo-métalliques comme activateur de réaction et donc de ne pouvoir être utilisés pour des formulations comprenant des composés organo-métalliques. On peut citer en exemple les composites polyesters polymérisés à basse température en présence d'octoate de cobalt dont l'utilisation principale est la fabrication de sanitaires, marbres synthétiques, gels coat, mastics et autres.

L'homme du métier se trouve alors contraint de ne pouvoir introduire des charges en quantité importante ou suffisamment fines pour une amélioration des propriétés esthétiques comme un abaissement du prix de revient.

Confronté à ce problème, la Demanderesse a recherché et mis au point un agent dispersant qui permet, de manière surprenante, l'introduction de forte quantité de charges minérales et/ou organiques aussi bien dans des composés, se transformant à basse température ou sous l'action de la chaleur et en présence ou non d'accélérateur à base de composés organométalliques, que dans toute autre résine polymère telle que par exemple les thermodurcissables composés entre autre des résines acryliques, phénoplastes, aminoplastes, époxydes, réactifs pour polyuréthannes réticulés, alkydes, polyesters insaturés produits par la réaction de condensation de l'anhydride maléique en présence ou non de dérivés phtaliques sur un alkylène glycol ou un polyalkylène glycol de bas poids moléculaire dans du styrène susceptible d'être copolymérisé avec la résine polyester ou telle que par exemple les thermoplastiques composés entre autre des polyéthylènes basse ou haute densités, linéaires ou ramifiés, des polypropylènes homopolymères ou copolymères, des polyisobutylènes et des copolymères obtenus par les combinaisons lors de la copolymérisation de deux au moins des monomères éthylène, propylène et butylène ou bien encore d'autres polyoléfines telles que par exemple des chlorures de polyvinyle, des polystyrènes ainsi que des polyoléfines thermoplastiques halogénées ou non, modifiées ou non par greffage ou copolymérisation telles que par exemple des polyéthylènes téréphtalate, des polyoléfines halogénées, des polyéthylènes vinylacétate, éthylène-acide acrylique, éthylène-éthylacrylate, éthylène-méthylacrylate, éthylène-butylacrylate ; des polypropylènes modifiés EPDM (éthylène-propylène-diène-monomère), des polypropylènes modifiés SEBS (styrène-éthylène-butadiène-styrène) et/ou copolymères précités par mélange physique.

La Demanderesse a également trouvé de manière surprenante que le remplacement des groupes phosphate, phosphonate, sulfate ou sulfonate dans les esters phosphatés ou phosphonés ou sulfatés ou sulfonés de l'art antérieur par le groupe anhydride organique apporté par des composés tels que par exemple l'anhydride pyromellitique, le tétrahydrofurane tétracarboxylique dianhydride, le cyclopentane tétracarboxylique dianhydride, le naphtalène tétracarboxylique dianhydride, ainsi que la présence de la fonction anhydride sur des substances présélectionnées permet d'obtenir des agents dispersants utilisables pour tout type de résines, qu'elle soient par exemple thermoplastiques, thermodurcissables, ou encore transformables à chaud ou à basse température.
Ainsi un des buts de l'invention est la mise au point de nouveaux agents dispersants de formule générale (I) : 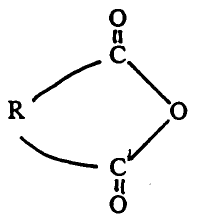
dans laquelle 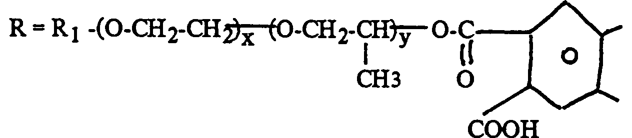
ou 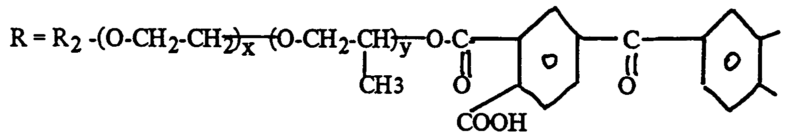
avec x et y des nombres allant de 0 à 100 inclus tels que la somme (x + y) varie de 0 à 100 inclus
et R₁ pouvant désigner un radical alkyle pouvant contenir jusqu'à 40 atomes de carbone si x ou y ne sont pas nuls et possédant bornes incluses de 10 à 40 atomes de carbone si x et y sont nuls, ou bien encore un radical aryle, alkylaryle, arylalkyle, polyaryle ramifié ou non, pouvant chacun comporter un ou plusieurs groupements fonctionnels du type carboxyle, ester, éther, nitro, amine tertiaire, amide, imide, nitrile, halogène ou uréthanne
et R₂ pouvant désigner un radical alkyle pouvant contenir jusqu'à 40 atomes de carbone quels que soient x et y, ou bien encore un radical aryle, alkylaryle, arylalkyle, polyaryle ramifié ou non, pouvant chacun comporter un ou plusieurs groupements fonctionnels du type carboxyle, ester, éther, nitro, amine tertiaire, amide, imide, nitrile, halogène ou uréthanne
permettant l'introduction de charges minérales et/ou organiques jusqu'à 90 % en poids par rapport aux compositions thermoplastiques, thermodurcissables ou bien encore transformables à basse température comme à chaud tout en conservant la fluidité et l'homogénéité des compositions ainsi que leur aptitude à la transformation ultérieure.

Un but supplémentaire de l'invention est de fournir des compositions thermoplastiques, thermodurcissables ou bien encore transformables à basse température comme à chaud conduisant à des matières plastiques à propriétés physico-chimiques telles que mécaniques, thermiques, diélectriques et esthétiques accrues.

Ces buts sont atteints grâce à l'ajout sur la charge minérale et/ou organique ou dans la composition avant l'introduction de la charge ou après, de l'agent selon l'invention de formule générale (I) : 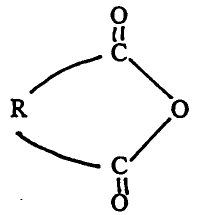
dans laquelle 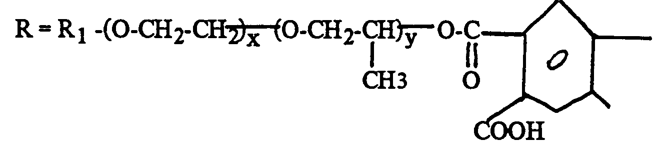
ou 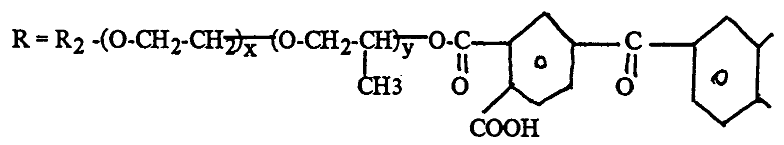
dans laquelle :
- x et y sont des nombres allant de 0 à 100 inclus tels que la somme (x + y) varie de 0 à 100 inclus
- R₁ est un radical alkyle pouvant contenir jusqu'à 40 atomes de carbone si x ou y ne sont pas nuls et possédant, bornes, incluses de 10 à 40 atomes de carbone si x et y sont nuls, ou bien encore un radical aryle, alkylaryle, arylalkyle, polyaryle ramifié ou non, pouvant chacun comporter un ou plusieurs groupements fonctionnels du type carboxyle, ester, éther, nitro, amine tertiaire, amide, imide, nitrile, halogène ou uréthanne
- et R₂ est un radical alkyle pouvant contenir jusqu'à 40 atomes de carbone quels que soient x et y ou bien encore un radical aryle, alkylaryle, arylalkyle, polyaryle ramifié ou non, pouvant chacun comporter un ou plusieurs groupements fonctionnels du type carboxyle, ester, éther, nitro, amine tertiaire, amide, imide, nitrile, halogène ou uréthanne.

A titre d'exemple on peut citer pour les radicaux R₁ ou R₂, les radicaux aussi divers que n-décyl, n-dodécyl, n-tétradécyl, n-hexadécyl, n-heptadécyl, n-octadécyl, et tous les radicaux alkyle linéaires ou ramifiés pouvant contenir jusqu'à 40 atomes de carbone, phényl, méthyl-2-, butyl-2-, phényl-3-, paranonylphényl, paraméthylphényl, le βnaphtyl, ou bien encore comme radical polyaryle des radicaux aussi divers que les di(phényl-1-éthyl) phénols communément appelés les distyrylphénols, les tri(phényl-1-éthyl) phénols communément appelés les tristyrylphénols, ou encore des produits de condensation par exemple de caprolactone ou d'oxyde d'alkylène ou également des oligomères du polystyrène ou des oligomères de copolymère de styrène avec un autre monomère.

Alors que l'art antérieur propose des compositions thermoplastiques ou thermodurcissables fortement chargées contenant des dispersants de type organo-phosphoré, la composition chargée selon l'invention s'en distingue par le fait qu'elle comprend essentiellement
(a) une résine thermoplastique choisie par exemple parmi des polyéthylènes basse ou haute densités, linéaires ou ramifiés, des polypropylènes homopolymères ou copolymères, des polyisobutylènes et des copolymères obtenus par les combinaisons lors de la copolymérisation de deux au moins des monomères éthylène, propylène et butylène ou bien encore d'autres polyoléfines telles que par exemple des chlorures de polyvinyle, des polystyrènes ainsi que des polyoléfines thermoplastiques halogénées ou non, modifiées ou non par greffage ou copolymérisation telles que par exemple des polyéthylènes téréphtalate des polyoléfines halogénées, des polyéthylènes vinylacétate, éthylène-acide acrylique, éthylène-éthylacrylate, éthylène-méthylacrylate, éthylène-butylacrylate, des polypropylènes modifiés EPDM (éthylènepropylène-diène-monomère), des polypropylènes modifiés SEBS (styrène-éthylène-butadiène-styrène) et/ou copolymères précités par mélange physique et plus particulièrement choisie parmi les polyéthylènes, les polypropylènes, les terpolymères butylène-propylène-éthylène, les chlorures de polyvinyle, les polystyrènes, les polyéthylènes vinylacétate, les EPDM
   ou bien une résine thermodurcissable choisie entre autre parmi des résines acryliques, phénoplastes, aminoplastes, époxydes, réactifs pour polyuréthannes, alkydes, polyesters insaturés produits par la réaction de condensation de l'anhydride maléique en présence ou non de dérivés phtaliques sur un alkylène glycol ou un polyalkylène glycol de bas poids moléculaire dans du styrène susceptible d'être copolymérisé avec la résine polyester et plus particulièrement choisie parmi les résines acryliques, les réactifs des polyuréthannes réticulés et les polyesters insaturés.
(b) jusqu'à 90 % en poids, par rapport au poids de la résine et de la charge, d'une ou plusieurs charges minérales et/ou organiques d'origines naturelles ou synthétiques généralement choisies parmi les sels et/ou oxydes minéraux tels que le carbonate de calcium naturel ou précipité, le carbonate de magnésium, le carbonate de zinc, la dolomie, la chaux, la magnésie, le sulfate de baryum, le sulfate de calcium, l'hydroxyde d'aluminium, la silice, la wollastonite, les argiles et autres silico-alumineux tels que le kaolin, le talc, le mica, les billes de verre pleines ou creuses, les oxydes métalliques tels que par exemple l'oxyde de zinc, les oxydes de fer, l'oxyde de titane, et plus particulièrement choisie parmi le carbonate de calcium naturel ou précipité, l'hydroxyde d'aluminium, l'oxyde et l'hydroxyde de magnésium, le sulfate de baryum et l'oxyde de titane. Toutes ces matières pulvérulentes minérales peuvent être mises en oeuvre seules ou en combinaisons. Peuvent y être adjointes des matières pulvérulentes organiques d'origine naturelle ou synthétique tels que par exemple les colorants, l'amidon, les fibres et la farine de cellulose, l'azodicarbonamide ou les fibres de carbone. Ces matières organiques peuvent également être mises en oeuvre seules ou en combinaison et sans matière minérale pulvérulente.
(c) un agent dispersant de formule générale (I) 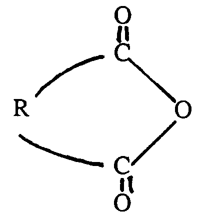 dans laquelle 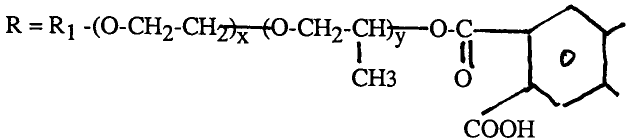 ou 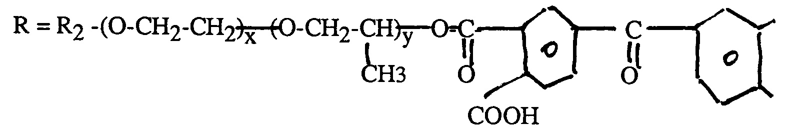 avec
   - x et y des nombres allant de 0 à 100 inclus tels que la somme (x + y) varie de 0 à 100 inclus
   - R₁ et R₂ deux radicaux différents ou identiques choisis parmi les radicaux alkyle pouvant contenir jusqu'à 40 atomes de carbone, aryle, alkylaryle, arylalkyle, polyaryle ramifié ou non, pouvant chacun comporter un ou plusieurs groupements fonctionnels du type carboxyle, ester, éther, nitro, amine tertiaire, amide, imide, nitrile, halogène ou uréthanne
      et particulièrement de 0,3 à 5 % en poids, par rapport à la charge, et plus particulièrement de 0,5 à 3 % en poids par rapport à la charge.
(d) éventuellement d'autres additifs de type connu choisis notamment parmi les catalyseurs de polymérisation ou de greffage, les stabilisants thermiques ou photo-chimiques, les antioxydants, les antiretraits, les antistatiques, les plastifiants, les lubrifiants, les agents de démoulage, les ignifugeants, les fibres et les billes de verre, les épaississants minéraux tels que l'hydroxyde de magnésium et autres.

Le procédé de fabrication des compositions polymères chargées, fluides et homogènes selon l'invention se caractérise en ce que l'agent dispersant selon l'invention est ajouté soit sur la charge minérale et/ou organique avant l'introduction dans la résine soit dans la résine avant l'introduction de la charge minérale et/ou organique soit dans la résine après l'introduction de la charge minérale et/ou organique.

Les compositions ainsi hautement chargées, fluides et homogènes, selon l'invention sont utilisées dans le cas par exemple de polyesters insaturés lors de la fa brication de pièces moulées selon l'une des techniques connues de moulage de feuilles ("sheet molding compound" ou SMC) ou de moulage dans la masse ("bulk molding compound" ou BMC) ou bien encore de polymérisation à basse température.

Elles peuvent être également utilisées dans la fabrication des thermodurcissables par exemple des mousses polyuréthannes rigides ou flexibles selon l'une quelconque des méthodes connues telles que "Slabstock" ou moulage, etc...

Elles peuvent être encore utilisées dans toutes les techniques de transformation des thermoplastiques telles que par exemple extrusion, injection, calandrage ou autres.

La portée et l'intérêt de l'invention seront mieux perçus grâce aux exemples suivants qui ne sauraient présenter aucun caractére limitatif.

### Exemple 1 :

Cet exemple concerne le mode opératoire de synthèse d'un agent dispersant selon l'invention.

### Essai 1 :

Ainsi, dans un bécher en inox de 800 ml muni d'un agitateur de laboratoire PENDRAULIK monté d'une pale de diamètre de 5 cm, on introduit la quantité de carbonate de propylène nécessaire à l'obtention d'un produit final à 50 % en poids de matière active, puis on agite le carbonate à 500 tours/minute tout en le chauffant à 50°C avant d'y dissoudre 1,1 moles d'anhydride pyromellitique.

Après dissolution complète de l'anhydride on introduit progressivement dans le bécher 1 mole du produit à faire réagir.

Dans cet essai, le produit à faire réagir est le tristyryphénol avec 60 motifs oxyde d'éthylène.

La réaction s'étant poursuivie sous agitation pendant une demi-heure après la fin de l'ajout, on cuit le produit de réaction pendant 2 heures à 70°C.

En fin de manipulation l'agent, selon l'invention, a un aspect liquide couleur jaune-orangé, un indice d'acide de 32,4 mg/g déterminé selon la norme NF T30-402 et une formule répondant à la formule générale (I) avec R₁ = radical tristyrylphénol, x = 60 et y = 0.

### Essai 2 :

Dans un bécher en inox de 800 ml muni d'un agitateur de laboratoire PENDRAULIK équipé d'une pale de diamètre de 5 cm, on introduit la quantité de carbonate de propylène nécessaire à l'obtention d'un produit final à 30 % en poids de matière active, puis on agite le carbonate à 500 tours/minute tout en le chauffant à 50°C avant d'y dissoudre 1,1 moles d'anhydride pyromellitique.

Après dissolution complète de l'anhydride on introduit progressivement dans le bécher 1 mole du produit à faire réagir.

Dans cet essai, le produit à faire réagir est le tristyryphénol avec 14 motifs oxyde d'éthylène et 2 motifs oxyde de propylène.

La réaction s'étant poursuivie sous agitation pendant une demi-heure après la fin de l'ajout, on cuit le produit de réaction pendant 2 heures à 70°C.

En fin de manipulation l'agent, selon l'invention, a un aspect liquide couleur jaune-orangé, un indice d'acide de 50 mg/g déterminé selon la norme NF T30-402 et une formule répondant à la formule générale (I) dans laquelle R₁ est le radical tristyrylphénol, x = 14 et y = 2.

### Essai 3 :

Dans les mêmes conditions opératoires et avec le même matériel que l'essai 2, on fait réagir 1 mole de tristyrylphénol avec 40 motifs d'oxyde d'éthylène sur 1,1 moles d'anhydride pyromellitique en présence de carbonate de propylène pour obtenir un agent, selon l'invention, de formule répondant à la formule générale (I) pour laquelle R₁ est un radical tristyrylphénol, x = 40 et y = 0, et ayant un aspect liquide couleur jaune-orangé ainsi qu'un indice d'acide de 28 mg/g déterminé selon la norme NF T30-402.

### Essai 4 :

Dans les mêmes conditions opératoires et avec le même matériel que l'essai 2, on fait réagir 1 mole de tristyrylphénol avec 60 motifs d'oxyde d'éthylène sur 1,1 moles d'anhydride pyromellitique en présence de carbonate de propylène pour obtenir un agent selon l'invention de formule répondant à la formule générale (I) pour laquelle R₁ est unradical tristyrylphénol, x = 60 et y = 0 et ayant un aspect liquide couleur jaune-orangé ainsi qu'un indice d'acide de 20 mg/g déterminé selon la norme NF T30-402.

### Essai 5 :

Dans les mêmes conditions opératoires et avec le même matériel que l'essai 2, on fait réagir 1 mole de tristyrylphénol avec 100 motifs d'oxyde d'éthylène sur 1,1 moles d'anhydride pyromellitique en présence de carbonate de propylène pour obtenir un agent selon l'invention de formule répondant à la formule générale (I) pour laquelle R₁ est un radical tristyrylphénol, x = 100 et y = 0 et ayant un aspect liquide couleur jaune-orangé ainsi qu'un indice d'acide de 11 mg/g déterminé selon la norme NF T30-402.

### Essai 6 :

Dans les mêmes conditions opératoires et avec le même matériel que l'essai 2, on fait réagir 1 mole de méthoxy-polyéthylène glycol de poids moléculaire égal à 2000 avec 1,1 moles d'anhydride pyromellitique en présence de carbonate de propylène pour obtenir un agent selon l'invention de formule répondant à la formule générale (I) pour laquelle R₁ est le groupe méthyle, x = 45, y = 0 et ayant un aspect liquide couleur jaune-orangé ainsi qu'un indice d'acide de 27 mg/g déterminé selon la norme NF T30-402.

### Essai 7 :

Dans un bécher en inox de 500 ml équipé d'un disperseur PENDRAULIK muni d'une pâle de 4 cm de diamètre on introduit sous agitation 91,06 g de tristyrylphénol oxyéthylé 14 fois et oxypropylé 2 fois puis on chauffe à 50°C avant d'ajouter dans le milieu 82,8 g d'anhydride pyromellitique. Après dissolution totale, on coule progressivement dans le bécher 334,3 g de tristyrylphénol oxyéthylé 14 fois et oxypropylé 2 fois puis on laisse réagir une demi-heure à 50°C avant de terminer la réaction par une cuisson à 70°C pendant 2 heures.

En fin de manipulation, l'agent selon l'invention, a un aspect pâteux, un indice d'acide de 111 mg/g déterminé selon la norme NF T30-402 et une formule répondant à la formule générale (I) avec R₁ = radical tristyrylphénol, x = 14 et y = 2.

### Essai 8 :

Dans un bécher en inox de 500 ml équipé d'un disperseur PENDRAULIK muni d'une pâle de 4 cm de diamètre on introduit sous agitation 70,6 g d'un alcool linéaire comprenant 16 à 18 atomes de carbone et commercialisé par la société CONDEA sous le nom de NALFOL 16-18 S puis on chauffe à 50°C avant d'ajouter dans le milieu 126 g d'anyhydride pyromellitique. Après dissolution totale, on coule progressivement dans le bécher 109,2 g de NALFOL 16-18 S puis on laisse réagir une demi-heure à 50°C avant de terminer la réaction par une cuisson à 70°C pendant 2 heures.

En fin de manipulation, l'agent selon l'invention, a un aspect cireux, un indice d'acide de 329 mg/g déterminé selon la norme NF T30-402 et une formule répondant à la formule générale (I) avec R₁ = radical alkyle possédant 16 à 18 atomes de carbone, x = 0 et y = 0.

### Essai 9 :

Dans un bécher en inox de 500 ml on introduit 108,9 g d'un alcool ramifié à 36 atomes de carbone commercialisé sous le nom ISOFOL C₃₆ par la société CONDEA puis on chauffe à 50°C tout en agitant à 500 tours/minute à l'aide d'un disperseur PENDRAULIK équipé d'une pâle de 4 cm de diamètre.

Ensuite on dissout dans le milieu 86,2 g d'anhydride pyromellitique.

Après dissolution totale, on coule progressivement 141 g d'ISOFOL C36 dans le bécher et on laisse réagir une demi-heure avant de cuire 2 heures à 70°C.

En fin de manipulation, l'agent selon l'invention, a un aspect solide, un indice d'acide de 165 mg/g déterminé selon la norme NF T30-402 et une formule répondant à la formule générale (I) avec R₁ = radical alkyle possédant 36 atomes de carbone, x = 0 et y = 0.

### Essai 10 :

Dans un bécher en inox de 500 ml on introduit 90,7 g d'un alcool linéaire à 10 atomes de carbone oxyéthylé 5 fois et commercialisé sous le nom de LAUROPAL 0205 par la société WITCO puis on chauffe à 50°C tout en agitant à 500 tours/minute à l'aide d'un disperseur PENDRAULIK équipé d'une pâle de 4 cm de diamètre.

Puis on dissout dans le milieu 108 g d'anhydride pyromellitique.

Après dissolution totale, on coule progressivement 124,8 g de LAUROPAL 0205 dans le bécher et on laisse réagir une demi-heure à 50°C avant de cuire 2 heures à 70°C.

En fin de manipulation, l'agent selon l'invention, a un aspect pâteux, un indice d'acide de 213 mg/g déterminé selon la norme NF T30-402 et une formule répondant à la formule générale (I) avec R₁ = radical alkyle possédant 10 atomes de carbone, x = 5 et y = 0.

### Essai 11 :

Dans un bécher en inox de 800 ml muni d'un agitateur de laboratoire PENDRAULIK équipé d'une pale de diamètre de 5 cm, on introduit la quantité de carbonate de propylène nécessaire à l'obtention d'un produit final à 30 % en poids de matière active, puis on agite le carbonate à 500 tours/minute tout en le chauffant à 50°C avant d'y dissoudre 1,1 moles d'anhydride pyromellitique.

Après dissolution complète de l'anhydride on introduit progressivement dans le bécher 1 mole de tristyrylphénol oxyéthylé 16 fois à faire réagir.

Dans cet essai, le produit à faire réagir est le produit de polymérisation de la caprolactone avec un polyaryl polyéther. Le produit obtenu étant un tristyrylphénol oxyéthylé 16 fois sur lequel a été condensé 2 motifs caprolactone.

La réaction s'étant poursuivie sous agitation pendant une demi-heure après la fin de l'ajout, on cuit le produit de réaction pendant 2 heures à 70°C.

En fin de manipulation l'agent, selon l'invention, a un aspect liquide de couleur jaune, un indice d'acide de 47 mg/g déterminé selon la norme NF T30-402 et une formule répondant à la formule générale (I) dans laquelle R₁ est un radical polyaryle possédant des fonctions ester et éther, x = 0 et y = 0.

### Essai 12 :

Dans un bécher en inox de 800 ml muni d'un agitateur de laboratoire PENDRAULIK équipé d'une pale de diamètre de 5 cm, on introduit la quantité de carbonate de propylène nécessaire à l'obtention d'un produit final à 30 % en poids de matière active, puis on agite le carbonate à 500 tours/minute tout en le chauffant à 60°C avant d'y dissoudre 1,1 moles de benzophénone de dianhydride tétracarboxylique.

Après dissolution complète du dianhydride on introduit progressivement dans le bécher 1 mole de tristyrylphénol oxyéthylé 16 fois.

La réaction s'étant poursuivie sous agitation pendant une demi-heure à 60°C après la fin de l'ajout, on cuit le produit de réaction pendant 2 heures à 80°C.

En fin de manipulation l'agent, selon l'invention a un aspect liquide de couleur jaune, un indice d'acide de 102 mg/g déterminé selon la norme NF T30-402 et une formule répondant à la formule générale (I) dans laquelle R₂ est un radical tristyrylphénol avec x = 16 et y = 0.

### Exemple 2 :

Cet exemple concerne la dispersion de carbonate de calcium dans une résine polyester insaturé polymérisable à froid et l'obtention d'une composition hautement chargée, fluide et homogène, à l'aide de divers agents dispersants.

- Préparation de la composition hautement chargée et mesure de l'efficacité des divers agents dispersants par la mesure de sa rhéologie.

Dans un pot métallique d'environ 500 ml muni d'un agitateur de laboratoire PENDRAULIK équipé d'une pale d'un diamètre de 5 cm, on introduit au repos 100 g d'une résine polyester insaturé polymérisable à basse température commercialisée par la société BASF sous le nom de PALATAL P4 puis 3 g d'agent dispersant à tester. On homogénéise alors le mélange en 30 secondes au moyen de l'agitateur avant d'ajouter en 10 minutes sous agitation 300 g de carbonate de calcium naturel commercialisé par la société OMYA S.A. sous le nom MILLICARB. On maintient l'agitation pendant 5 minutes. Après les 15 minutes de durée du mélange, cette composition est conditionnée à 30°C pendant 2 heures et pendant 24 heures avant d'en mesurer la viscosité Brookfield à 30°C à l'aide d'un viscosimètre Brookfield type HBT et sous différents taux de cisaillements (5 T/mn, 10 T/mn, 20 T/mn, 50 T/mn). Ce mode opératoire a été utilisé dans les essais suivants pour tester les différents agents dispersants.

### Essai 13 :

Pas de dispersant utilisé.

### Essai 14 :

L'agent dispersant utilisé est un agent selon l'art antérieur, commercialisé par la société BYK, sous le nom BYK W 965.

### Essai 15 :

L'agent dispersant utilisé est un agent selon l'art antérieur, commercialisé par la société BYK, sous le nom BYK W 980.

### Essai 16 :

L'agent dispersant est l'agent selon l'invention de l'essai 1.

Les résultats obtenus sont rassemblés dans le tableau 1 suivant :

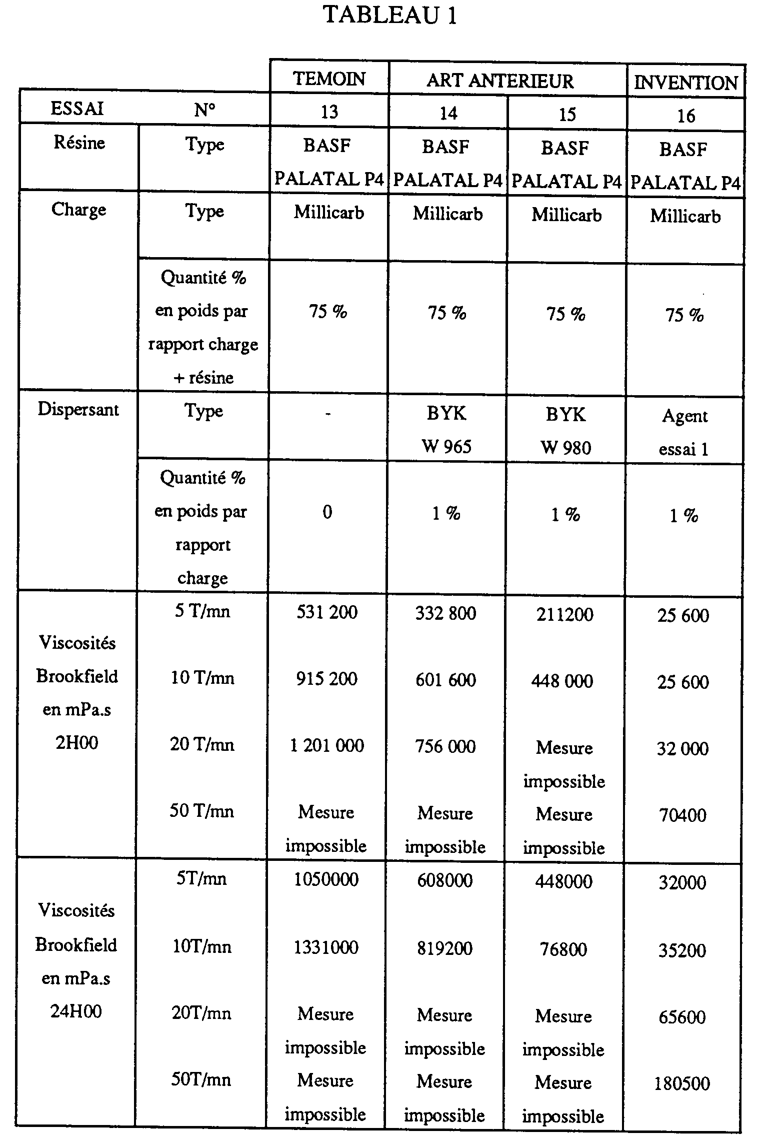

La lecture du tableau 1 permet de constater que la composition thermodurcissable obtenue au moyen de l'agent dispersant de formule générale (I) selon l'invention a des viscosités Brookfield minimales quel que soit le taux de cisaillement de la mesure de viscosité et est la seule à ne pas posséder de seuil d'écoulement. L'agent dispersant selon l'invention est donc l'agent le plus efficace et celui qui facilite le plus le travail du mélange à tous les stades de la fabrication.

Dans les mêmes conditions opératoires et avec le même matériel que ceux des essais 12 à 16, on a mélangé 250 g du même carbonate de calcium dénommé MILLICARB dans 100 g d'une résine polyester insaturée commercialisée par la société CRAY VALLEY sous le nom de NORSODYNE I 2984 V avec diverses quantités de dispersant à tester.

Les différents essais sont :

### Essai 17 :

Essai témoin sans utilisation de dispersant.

### Essai 18 :

Essai utilisant 1 % en poids, par rapport à la charge, de l'agent dispersant selon l'art antérieur commercialisé par la société BYK sous la dénomination BYK W980.

### Essai 19 :

Essai utilisant 1 % en poids, par rapport à la charge, d'un agent dispersant selon l'invention correspondant à l'agent dispersant de l'essai 1, c'est-à-dire répondant à la formule générale (I) ayant pour groupe R₁ le radical tristyrylphénol et avec x = 60 et y = 0.

### Essai 20 :

Essai utilisant 1 % en poids, par rapport à la charge, d'un agent dispersant selon l'invention correspondant à l'agent dispersant de l'essai 4, c'est-à-dire répondant à la formule générale (I) ayant pour groupe R₁ le radical tristyrylphénol et avec x = 60 et y = 0.

### Essai 21 :

Essai utilisant 1 % en poids, par rapport à la charge, d'un agent dispersant selon l'invention correspondant à l'agent dispersant de l'essai 2, c'est-à-dire répondant à la formule générale (I) ayant pour groupe R₁ le radical tristyrylphénol et avec x = 14 et y = 2.

### Essai 22 :

Essai utilisant 1 % en poids, par rapport à la charge, d'un agent dispersant selon l'invention correspondant à l'agent dispersant de l'essai 3, c'est-à-dire répondant à la formule générale (I) ayant pour groupe R₁ le radical tristyrylphénol et avec x = 40 et y = 0

### Essai 23 :

Essai utilisant 1 % en poids, par rapport à la charge, d'un agent dispersant selon l'invention correspondant à l'agent dispersant de l'essai 5, c'est-à-dire répondant à la formule générale (I) ayant pour groupe R₁ le radical trystyrylphénol et avec x = 100 et y = 0.

### Essai 24 :

Essai utilisant 1 % en poids, par rapport à la charge, d'un agent dispersant selon l'invention correspondant à l'agent dispersant de l'essai 6, c'est-à-dire répondant à la formule générale (I) ayant pour groupe R₁ le radical méthyle et avec x = 45 et y = 0.

### Essai 25 :

Essai utilisant 1 % en poids, par rapport à la charge, d'un agent dispersant selon l'invention correspondant à l'agent dispersant de l'essai 11, c'est-à-dire répondant à la formule générale (I) ayant pour radical R₁ le radical polyaryl à fonction ester et éther avec x = 0 et y = 0.

### Essai 26 :

Essai utilisant 1 % en poids, par rapport à la charge, d'un agent dispersant selon l'invention correspondant à l'agent dispersant de l'essai 12, c'est-à-dire répondant à la formule générale (I) ayant pour radical R₂ le radical tristyrylphénol avec x = 16 et y = 0.

### Essai 27 :

Essai utilisant, 0,5 % en poids, par rapport à la charge, de l'agent dispersant de l'essai 4 c'est-à-dire répondant selon l'invention à la formule (I) ayant pour radical R₁ le radical tristyrylphénol avec x = 60 et y = 0.

### Essai 28 :

Essai utilisant, 1,5 % en poids, par rapport à la charge, de l'agent dispersant de l'essai 4, selon l'invention.

### Essai 29 :

Essai utilisant, 2 % en poids, par rapport à la charge, de l'agent dispersant de l'essai 4, selon l'invention.

### Essai 30 :

Essai utilisant, 3 % en poids, par rapport à la charge, de l'agent dispersant de l'essai 4, selon l'invention.

### Essai 31:

Essai utilisant, 5 % en poids, par rapport à la charge, de l'agent dispersant de l'essai 4, selon l'invention.

Les résultats obtenus pour les mesures de viscosité Brookfield à différents taux de cisaillement (5 T/mn, 10 T/mn, 20 T/mn, 50 T/mn, 100 T/mn) sont rassemblés dans les tableaux 2 et 2 (suite) suivants :

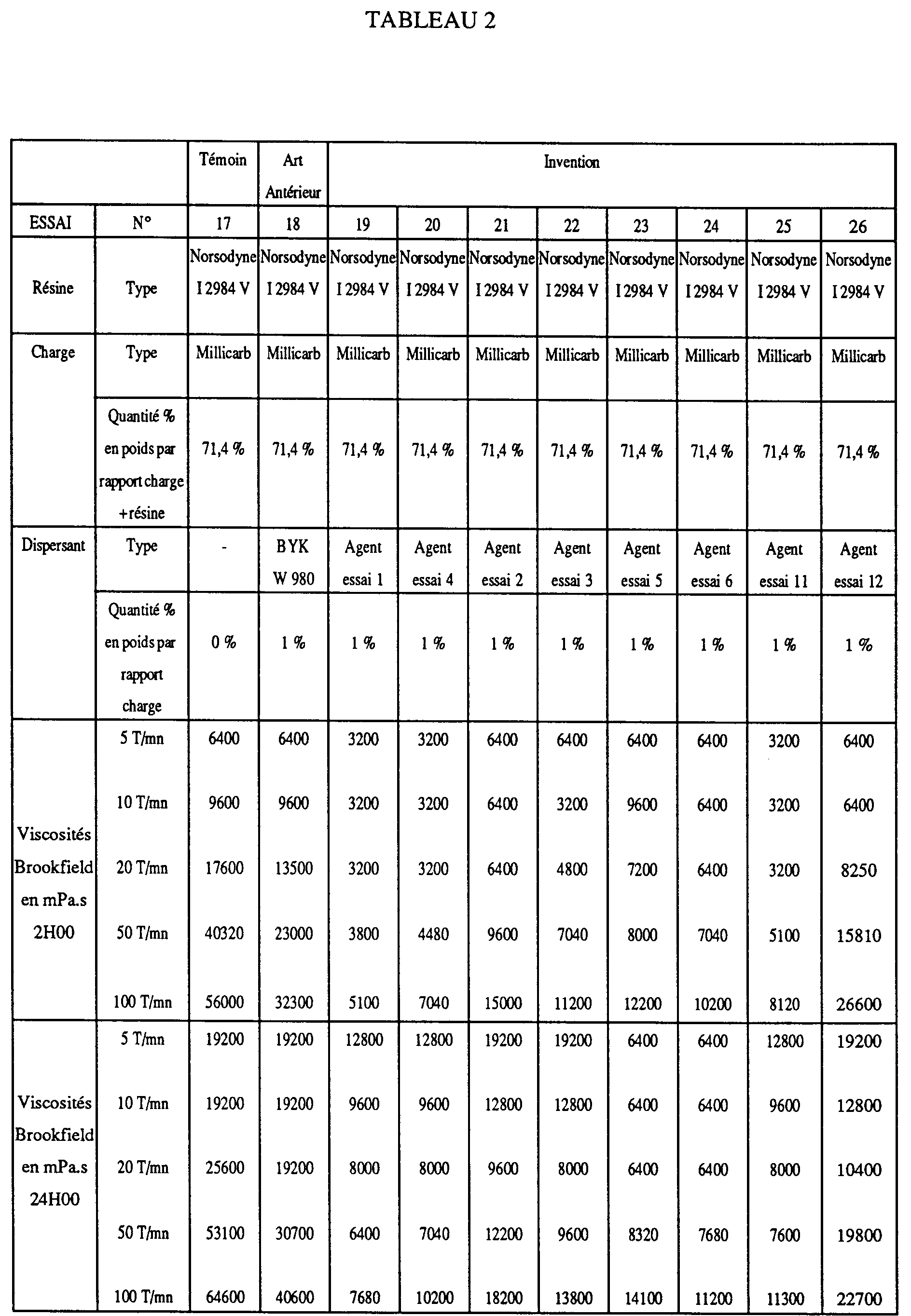

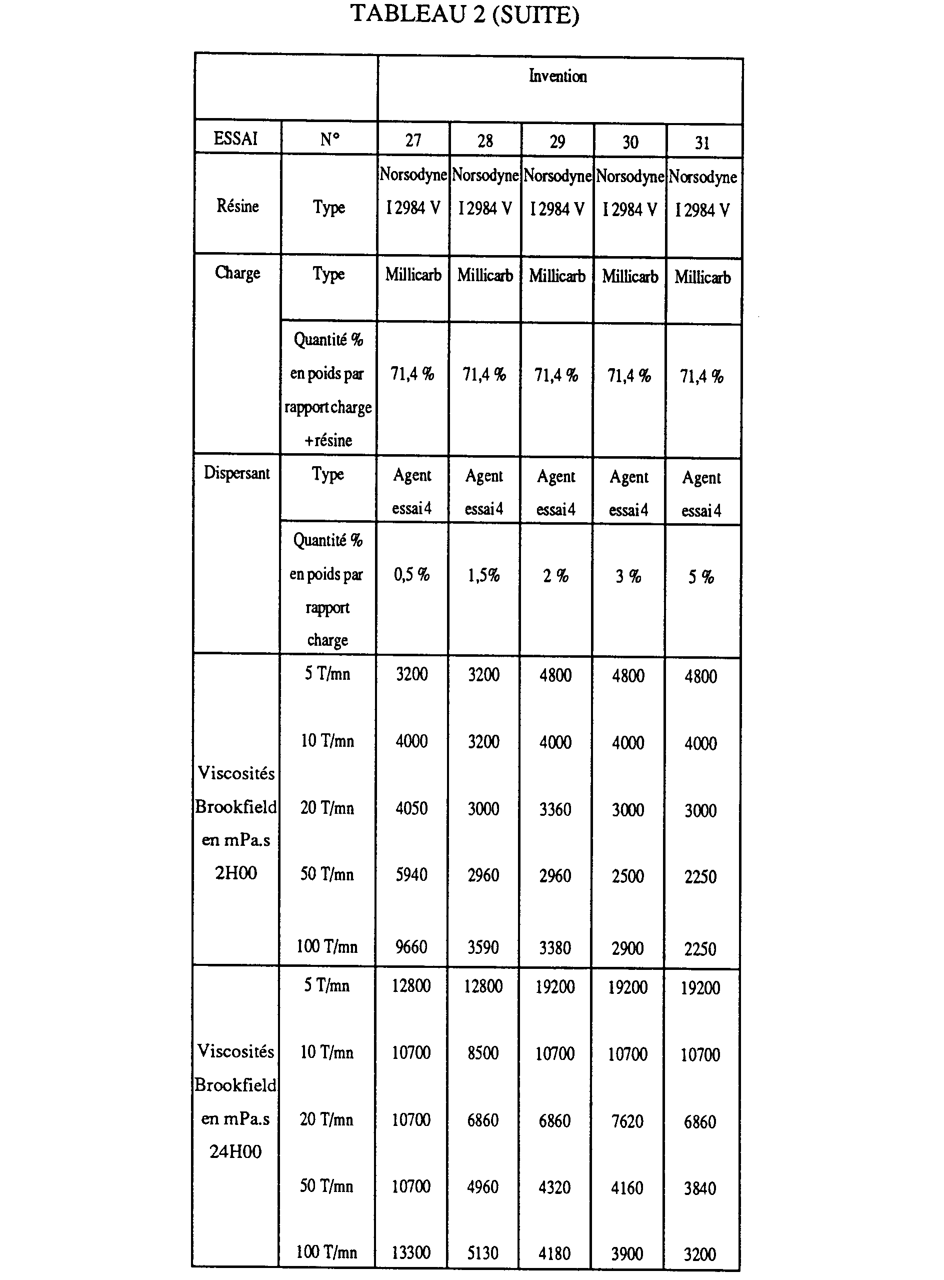

La lecture des tableaux 2 et 2 (suite) permet de constater que la composition thermodurcissable obtenue au moyen de l'agent dispersant de formule générale (I) selon l'invention a des viscosités Brookfield minimales quel que soit le taux de cisaillement de la mesure de la viscosité et permet donc d'évaluer l'efficacité accrue des agents dispersants selon l'invention par rapport au témoin ou à l'art antérieur.

### Exemple 3 :

Cet exemple concerne la polymérisation à froid de composition polyesters insaturés utilisant un système catalytique à base d'un dérivé de cobalt.

Ainsi dans un pot métallique d'environ 500 ml muni d'un agitateur de laboratoire PENDRAULIK équipé d'une pâle d'un diamètre de 5 cm, on introduit au repos 1,5 g d'agent dispersant à tester, 150 g d'une résine polyester insaturé polymérisable à basse température commercialisée par la société BASF sous le nom de PALATAL P4 puis 3 g d'octoate de Cobalt titrant 1 % en Cobalt et commercialisé par la société PERGAN sous le nom de PERGAQUICK C12. On homogénéise alors le mélange en 1 minute au moyen de l'agitateur avant d'ajouter en 6 minutes sous agitation 150 g de carbonate de calcium naturel commercialisé par la société OMYA S.A. sous le nom de CALIBRITE. On maintient l'agitation pendant 5 minutes. Après les 11 minutes de durée du mélange, on ajoute 1,8 g d'initiateur de réaction commercialisé par la société PERGAN sous le nom de PEROXAN MESOL. Cet instant d'ajout correspond au départ de la réaction de polymérisation. L'évolution de la polymérisation est suivie par la mesure de la température de la réaction à l'aide d'un enregistreur du type LINESIS L250 équipée d'un thermocouple commercialisé par la société PROLABO sous la référence type K préalablement calibré à 0°C et 100°C.

La polymérisation caractérisée par une exothermie est jugée terminée lorsque la température diminue et complète si au bout d'une heure maximum, après l'ajout de l'initiateur, la composition finale a durci.

Les différents agents dispersants testés sont :

### Essai 32 :

Agent selon l'art antérieur commercialisé par la société BYK sous le nom de BYK W 995.

### Essai 33 :

Agent selon l'art antérieur commercialisé par la société BYK sous le nom de BYK W 996.

### Essai 34 :

Agent selon l'invention correspondant à l'agent dispersant de l'essai 2 c'est-à-dire répondant à la formule générale (I) ayant pour groupe R₁ le radical tristyrylphénol, x = 14 et y = 2.

### Essai 35 :

Agent selon l'invention correspondant à l'agent dispersant de l'essai 5, c'est-à-dire répondant à la formule générale (I) ayant pour groupe R₁ le radical tristyrylphénol, x = 100 et y = 0.

### Essai 36 :

Agent selon l'invention correspondant à l'agent dispersant de l'essai 6, c'est-à-dire répondant à la formule générale (I) ayant pour groupe R₁ le radical méthyle et x = 45 et y = 0

Les résultats obtenus figurent dans le tableau 3 suivant :

**TABLEAU 3**

| | ART ANTERIEUR | | INVENTION | | |
|---|---|---|---|---|---|
| ESSAI N° | 32 | 33 | 34 | 35 | 36 |
| Dispersant type | BYK W 995 | BYK W 996 | Agent essai 2 | Agent essai 5 | Agent essai 6 |
| Température maximale atteinte par exothermie (°C) | Pas d'exothermie | Pas d'exothermie | 86,8 | 108,0 | 70,0 |
| Temps de durcissement | Pas de durcissement | Pas de durcissement | 43 mn | 34 mn | 47 mn |

La lecture du tableau 3 permet de constater que les réactions de polymérisation à froid de composition polyesters insaturés utilisant un système à base d'un dérivé de cobalt ne sont possibles qu'en présence des agents dispersants de formule générale (I) selon l'invention, alors qu'en présence d'agent dispersant de l'art antérieur aucune polymérisation n'a lieu.

### Exemple 4 :

Cet exemple concerne la dispersion de carbonate de calcium dans une résine type polystyrène, la mesure de l'efficacité de l'agent dispersant selon l'invention et l'obtention d'une composition chargée, fluide et homogène.

Dans ce but, la fabrication de la composition s'effectue par l'introduction dans un malaxeur GUITTARD à bras en Z préchauffé à 200°C, de 300 g de carbonate de calcium naturel commercialisé par la société OMYA S.A. sous le nom de MILLICARB pouvant être traité par l'ajout de 3 g de dispersant à tester.

Après maintien sous agitation à 12 tours/mn de la charge traitée ou non pendant 15 minutes, on introduit dans la chambre du malaxeur 200 g de polystyrène commercialisé par la société ATOCHEM sous le nom de LACQRENE 7240 de grade 4 ainsi que 0,5 g de stabilisant vendu par la société CIBA-GEIGY sous la dénomination IRGANOX 1010 et 3 g du dispersant à tester dans le cas où la charge n'a pas été traitée au préalable.

Le mélange réalisé et après 10 minutes à 12 tours/mn, on augmente successivement la vitesse d'agitation du malaxeur à 47 tours/mn pendant 10 minutes et 76 tours/mn pendant les 10 minutes suivantes.

Le compound est alors réalisé et l'efficacité de l'agent dispersant, selon l'invention, est déterminée par la mesure de l'indice de fluidité ou MFI du compound fabriqué et la comparaison de la valeur obtenue à celle d'une résine témoin ne contenant pas d'agent dispersant.

Selon la norme ASTM 1238, l'indice de fluidité ou MFI est la quantité de polymère et/ou copolymère qui s'écoule, exprimée en grammes pour 10 minutes, à une température choisie dans l'intervalle limité par les températures de ramollissement et de transformation sous une charge donnée normalisée (2,16 kg, 5 kg, 10 kg, 21,6 kg) au travers d'une filière de diamètre déterminé (2,09 millimètres à 2,10 millimètres) pendant un temps mesuré.

Cette mesure de MFI évaluée selon cette norme ASTM 1238 méthode A, a été effectuée à 200°C sous une charge de 5 kg après calendrage des compositions sous forme de plaques découpées en petits cubes de 2 millimètres de côté à l'aide d'un appareillage commercialisé par la société ZWICK de type 4105 pour les essais suivants:

### Essai 37 :

Composition témoin comprenant aucun agent dispersant.

### Essai 38 :

Composition, selon l'invention, contenant 1 % en poids, par rapport à la charge, d'un agent dispersant, selon l'invention et correspondant à l'agent de l'essai 7.

### Essai 39 :

Composition, selon l'invention, contenant 2 % en poids, par rapport à la charge, d'un agent dispersant, selon l'invention préalablement déposé à la surface de la charge utilisée et correspondant à l'agent de l'essai 7.

Les résultats obtenus sont consignés dans le tableau 4 ci-dessous.

**TABLEAU 4**

| | | TEMOIN | INVENTION | | |
|---|---|---|---|---|---|
| ESSAI N° | | 37 | 38 | 39 | |
| Résine Type | | LACQRENE 7240 | LACQRENE 7240 | LACQRENE 7240 | |
| Charge | Type | MILLICARB | MILLICARB | MILLICARB | |
| | Quantité % en poids par rapport charge + résine | 60 % | 60 % | 60 % | |
| Dispersant | Type | - | Agent essai 7 | Agent essai 7 | |
| | Quantité % en poids par rapport charge | 0 % | 1 % | 2 % | |
| MFI (g/10 mn) 200/5 | | 0,7 | 2,0 | 3,0 | |

La lecture du tableau 4 permet de constater l'augmentation de la valeur de l'indice de fluidité (MFI) des compositions selon l'invention, c'est-à-dire des compositions contenant l'agent dispersant de formule générale (I) selon l'invention et donc permet de constater que les compositions selon l'invention sont les plus fluides.

### Exemple 5:

Cet exemple concerne la dispersion d'hydroxyde de magnésium dans un mélange-maître de polypropylène à l'aide d'un agent dispersant selon l'invention, la mesure de l'efficacité de l'agent dispersant selon l'invention et l'obtention d'une composition chargée, fluide et homogène.

Dans ce but, la fabrication du mélange-maître s'effectue dans chacun des essais suivants par l'introduction, dans un malaxeur GUITTARD à bras en Z préchauffé à 230°C, de 300 g d'hydroxyde de magnésium commercialisé par la société MARTINSWERKE sous le nom de MAGNIFIN H5 suivi après maintien sous agitation à 12 tours/mn de la charge pendant 15 minutes, de l'introduction dans la chambre du malaxeur de 200 g de polypropylène commercialisé par la société APPRYL sous le nom de LAQTENE 3120 MN1 ainsi que 0,5 g de stabilisant vendu par la société CIBA-GEIGY sous la dénomination IRGANOX 1010 et 3 g du dispersant à tester.

Le mélange réalisé et après 10 minutes à 12 tours/mn, on augmente successivement la vitesse d'agitation du malaxeur à 47 tours/mn pendant 10 minutes et 76 tours/mn pendant les 5 minutes suivantes.

Le mélange-maître est alors réalisé et l'efficacité de l'agent dispersant, selon l'invention, est déterminée par la mesure du MFI du compound fabriqué et la comparaison de la valeur obtenue à celle d'une résine témoin ne contenant pas d'agent dispersant.

Ces mesures de MFI effectuées selon le même mode opératoire, avec le même appareillage que celles de l'exemple 4 mais à une température de 230°C et sous une charge de 2,16 kg donnent, pour les essais suivants:

### Essai 40 :

Aucun agent dispersant dans le mélange-maître témoin.

### Essai 41 :

Composition, selon l'invention, contenant 1 % en poids, par rapport à la charge, d'un agent dispersant selon l'invention et correspondant à l'agent de l'essai 8.

les résultats répertoriés dans le tableau 5 ci-dessous :

**TABLEAU 5**

| | | TEMOIN | INVENTION | |
|---|---|---|---|---|
| ESSAI N° | | 40 | 41 | |
| Résine Type | | LAQTENE 3120 MIN1 | LAQTENE 3120 MIN1 | |
| Charge | Type | MAGNIFIN H5 | MAGNIFIN H5 | |
| | Quantité % en poids par rapport charge + résine | 60 % | 60 % | |
| Dispersant | Type | - | Agent essai 8 | |
| | Quantité % en poids par rapport charge | 0 % | 1 % | |
| MFI (g/10 mn) 230/2,16 | | 2,0 | 8,2 | |

La lecture du tableau 5 ci-dessus permet de constater la grande augmentation de l'indice de fluidité (MFI), donc la grande augmentation de fluidité de la résine polypropylène chargée avec de l'hydroxyde de magnésium au moyen d'un agent dispersant de formule générale (I) selon l'invention par rapport à une résine témoin ne contenant pas d'agent selon l'invention.

### Exemple 6 :

Cet exemple concerne la dispersion de carbonate de calcium dans une résine polyoléfine à l'aide d'un agent dispersant selon l'invention, la mesure de l'efficacité de l'agent dispersant selon l'invention et l'obtention d'une composition chargée, fluide et homogène.

Dans ce but, la fabrication de la composition s'effectue dans chacun des essais suivants par l'introduction, dans un mélangeur rapide GUEDU préchauffé pendant 2 heures à 140°C, de 841 g de carbonate de calcium naturel commercialisé par la société OMYA SPA. sous le nom de OMYACARB 2AV ainsi que de 149 g de résine polyoléfinique de type copolymère éthylène-propylène-butylène (EPB) et enfin de 8,4 g de dispersant à tester. Après maintien pendant 18 minutes de l'agitation, on procède au dégazage avant d'ouvrir le mélangeur pour en sortir la composition.

L'efficacité du dispersant est déterminée par la mesure de l'indice de fluidité effectuée dans les mêmes conditions et avec le même appareillage que dans les exemples 4 ou 5 mais à une température de 150°C et sous une charge de 5 kg.

Les différents agents testés correspondent aux essais suivants :

### Essai 42 :

Essai témoin ne contenant aucun agent dispersant.

### Essai 43 :

Essai utilisant 1 % en poids, par rapport à la charge, d'un agent dispersant selon l'art antérieur constitué d'un mélange de phosphate mono et diester de l'alcool myristique.

### Essai 44 :

Essai utilisant 1 % en poids, par rapport à la charge, d'un agent dispersant selon l'invention correspondant à l'agent de l'essai 8 c'est-à-dire répondant à la formule générale (I) ayant pour groupe R₁ le radical alkyle possédant 16 à 18 atomes de carbone, x = 0 et y = 0.

### Essai 45 :

Essai utilisant 1 % en poids, par rapport à la charge, d'un agent dispersant selon l'invention correspondant à l'agent de l'essai 9 c'est-à-dire répondant à la formule générale (I) ayant pour radical R₁ un radical alkyle possédant 36 atomes de carbone, x = 0 et y = 0.

Tous ces essais ont donné pour résultats, les résultats consignés dans le tableau 6 ci-dessous.

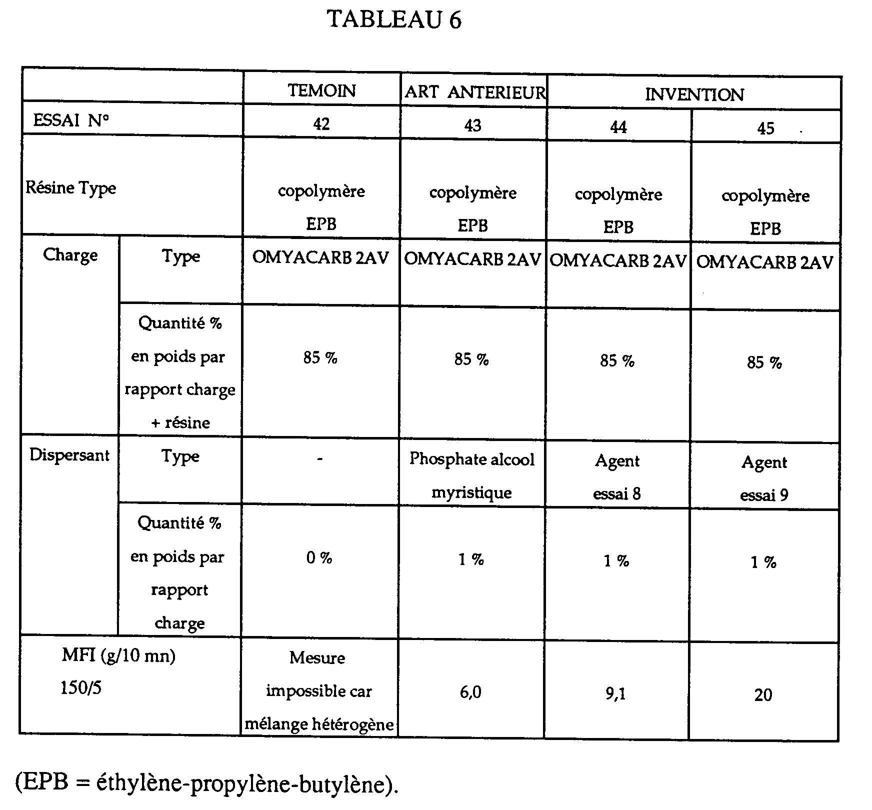

La lecture du tableau 6 ci-dessus permet de constater l'augmentation de MFI donc de fluidité des résines thermoplastiques chargées selon l'invention par rapport aux résines témoin ou de l'art antérieur et donc d'évaluer la plus grande efficacité des agents dispersants de formule générale (I) selon l'invention par rapport au témoin ou à l'art antérieur.

### Exemple 7 :

Cet exemple concerne la dispersion de carbonate de calcium et d'une résine type PVC dans un plastifiant à l'aide d'un agent dispersant selon l'invention, la mesure de l'efficacité de l'agent dispersant selon l'invention et l'obtention d'une composition chargée, fluide et homogène.

Dans ce but, dans un pot métallique d'environ 500 ml surmonté d'un agitateur de laboratoire PENDRAULIK, équipé d'une pâle d'un diamètre de 5 cm, on introduit au repos 80 g de plastifiant Dioctylphtalate commercialisé par la société ATOCHEM puis 1 g de stabilisant à base de baryum et de zinc commercialisé par la société ATOCHEM et 1 g d'agent dispersant à tester. On homogénéise alors le mélange en 30 secondes au moyen de l'agitateur avant d'ajouter en 5 minutes sous agitation 100 g de PVC commercialisé par la société ATOCHEM sous le nom PB 1302 et 100 g de carbonate de calcium naturel commercialisé par la société OMYA S.A. sous le nom de MILLICARB. On maintient l'agitation pendant 8 minutes. Cette composition est conditionnée à 23°C pendant 2 heures et pendant 24 heures avant d'en mesurer la viscosité Brookfield à 23°C à l'aide d'un viscosimètre Brookfield type HBT et sous différents taux de cisaillements (5 T/mn, 10 T/mn, 20 T/mn, 50 T/mn, 100 T/mn).

Les différents agents testés correspondent aux essais suivants :

### Essai 46 :

Essai témoin ne contenant aucun agent dispersant.

### Essai 47 :

Essai utilisant 1 % en poids, par rapport à la charge, d'un agent dispersant selon l'art antérieur constitué d'un phosphate de décanol oxyéthylé 5 fois.

### Essai 48 :

Essai utilisant 1 % en poids, par rapport à la charge, d'un agent dispersant selon l'invention correspondant à l'agent de l'essai 10 c'est-à-dire répondant à la formule générale (I) ayant comme radical R₁ un radical possédant 10 atomes de carbones, x = 5 et y = 0.

### Essai 49 :

Essai utilisant 2 % en poids, par rapport à la charge, d'un agent dispersant selon l'invention correspondant à l'agent de l'essai 10 c'est-à-dire répondant à la formule générale (I) ayant comme radical R₁ un radical possédant 10 atomes de carbones, x = 5 et y = 0.

Les résultats de ces essais figurent dans le tableau 7 ci-dessous.

**TABLEAU 7**

| | | Témoin | Art Antérieur | Invention | | |
|---|---|---|---|---|---|---|
| ESSAI | N° | 46 | 47 | 48 | 49 | |
| Résine | Type | ATOCHEM PB 1302 | ATOCHEM PB 1302 | ATOCHEM PB 1302 | ATOCHEM PB 1302 | |
| Charge | Type | Millicarb | Millicarb | Millicarb | Millicarb | |
| | Quantité % en poids par rapport charge + résine | 50 % | 50 % | 50 % | 50 % | |
| Dispersant | Type | - | Phosphate de décanol oxyéthylé | Agent | Agent | |
| | | | 5 fois | essai 10 | essai 10 | |
| | Quantité % en poids par rapport charge | 0 % | 1 % | 1 % | 2 % | |
| Viscosités Brookfield mPa.s 2H00 | 5 T/mn | 102400 | 25600 | 19200 | 19200 | |
| | 10 T/mn | 67200 | 19200 | 12800 | 12800 | |
| | 20 T/mn | 44800 | 14400 | 9600 | 9600 | |
| | 50 T/mn | 28800 | 10900 | 7680 | 7680 | |
| | 100 T/mn | 22080 | 8960 | 7040 | 7360 | |
| Viscosités Brookfield mPa.s 24H00 | 5 T/mn | 102400 | 25600 | 19200 | 19200 | |
| | 10 T/mn | 67200 | 19200 | 12800 | 12800 | |
| | 20 T/mn | 44800 | 14400 | 11200 | 9600 | |
| | 50 T/mn | 30080 | 10880 | 8960 | 8320 | |

La lecture du tableau 7 permet de constater qu'une résine type PVC chargée selon l'invention à l'aide d'agents dispersants de formule générale (I) selon l'invention a des viscosités Brookfield minimales quel que soit le taux de cisaillement et permet donc d'évaluer l'efficacité accrue des agents dispersants selon l'invention par rapport au témoin ou à l'art antérieur.

## Revendications

**1/** Composition polymère chargée, fluide et homogène caractérisée en ce qu'elle comprend essentiellement :
a) soit une résine thermoplastique choisie parmi les résines polyéthylènes basse ou haute densités, linéaires ou ramifiés, les polypropylènes homopolymères ou copolymères, les polyisobutylènes et les copolymères obtenus par les combinaisons lors de la copolymérisation de deux au moins des monomères éthylène, propylène et butylène ou bien encore les chlorures de polyvinyle, les polystyrènes ainsi que les polyoléfines halogénées ou non, modifiées ou non par greffage ou copolymérisation telles les polyéthylènes téréphtalate, les polyoléfines halogénées, les polyéthylènes vinylacétate, éthylène-acide acrylique, éthylène-éthylacrylate, éthylène-méthylacrylate, éthylène-butylacrylate ; les polypropylènes modifiés EPDM (éthylène-propylène diène monomère), les polypropylènes modifiés SEBS (styrène-éthylène butadiènes styrène) et/ou copolymères précités par mélange physique.
soit une résine thermodurcissable choisie parmi les résines acryliques, phénoplastes, aminoplastes, époxydes, réactifs pour polyuréthannes, alkydes, polyesters insaturés produits par la réaction de condensation de l'anhydride maléique en présence ou non de dérivés phtaliques sur un alkylène glycol ou un polyalkylène glycol de bas poids moléculaire dans du styrène susceptible d'être copolymérisé avec la résine polyester
b) jusqu'à 90 % en poids, par rapport au poids de la résine et de la charge, d'une ou plusieurs charges minérales et/ou organiques d'origines naturelles ou synthétiques choisies parmi les sels et/ou oxydes minéraux tels que le carbonate de calcium naturel ou précipité, le carbonate de magnésium, le carbonate de zinc, la dolomie, la chaux, la magnésie, le sulfate de baryum, le sulfate de calcium, l'hydroxyde d'aluminium, la silice, la wollastonite, les argiles et autres silico-alumineux tels que le kaolin, le talc, le mica, les billes de verre pleines ou creuses, les oxydes métalliques tels que par exemple l'oxyde de zinc, les oxydes de fer, l'oxyde de titane ou choisies parmi les matières organiques d'origine naturelle ou synthétique tels que les colorants, l'amidon, les fibres et la farine de cellulose, l'azodicarbonamide, les fibres de carbone ou leurs mélanges
c) un agent dispersant de formule (I) 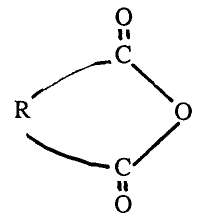 dans laquelle 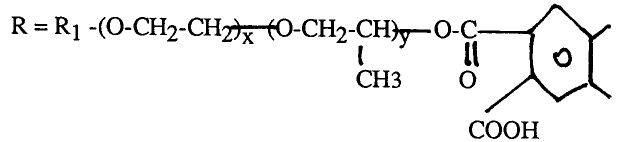 ou 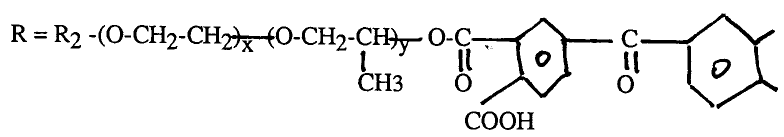 avec
x et y des nombres allant de 0 à 100 inclus tels que x + y varie de 0 à 100 inclus.
R₁ et R₂ deux radicaux différents ou identiques choisis parmi les radicaux alkyle pouvant contenir jusqu'à 40 atomes de carbone, aryle, alkylaryle, arylalkyle, polyaryle ramifié ou non pouvant chacun comporter un ou plusieurs groupements fonctionnels du type carboxyle, ester, éther, nitro, amine tertiaire, amide, imide, nitrile, halogène ou uréthanne
d) éventuellement d'autres additifs de type connu choisis parmi les catalyseurs de polymérisation ou de greffage, les stabilisants thermiques ou photochimiques, les antioxydants, les antiretraits, les antistatiques, les plastifiants, les lubrifiants, les agents de démoulage, les ignifugeants, les fibres et les billes de verre, les épaississants minéraux tels que l'hydroxyde de magnésium

**2/** Composition polymère chargée, fluide et homogène selon la revendication 1 caractérisée en ce que la résine thermoplastique est du type polyéthylène, polypropylène, terpolymère butylène-propylène-éthylène, chlorure de polyvinile, polystyrène, polyéthylène vinylacétate EPDM.

**3/** Composition thermodurcissable chargée, fluide et homogène selon la revendication 1 caractérisée en ce que la résine thermodurcissable est du type acrylique, réactif pour polyuréthanne, polyester insaturé.

**4/** Composition polymère chargée, fluide et homogène selon l'une quelconque des revendications 1 à 3 caractérisée en ce que la ou les charges minérales pulvérulentes sont le carbonate de calcium naturel ou précipité, l'hydroxyde d'aluminium, l'oxyde ou l'hydroxyde de magnésium, le sulfate de baryum ou l'oxyde de titane ou leurs mélanges.

**5/** Composition polymère chargée, fluide et homogène selon l'une quelconque des revendications 1 à 4 caractérisée en ce qu'elle contient de 0,3 % à 5 % en poids, par rapport à la charge, d'agent dispersant de formule générale (I).

**6/** Composition polymère chargée, fluide et homogène selon l'une quelconque des revendications 1 à 4 caractérisée en ce qu'elle contient de 0,5 % à 3 % en poids, par rapport à la charge, d'agent dispersant de formule générale (I).

**7/** Agent dispersant de charges minérales et/ou organiques pour la préparation des compositions polymères selon l'une quelconque des revendications 1 à 6 caractérisé en ce qu'il a pour formule générale, la formule (I) 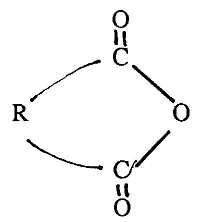 dans laquelle 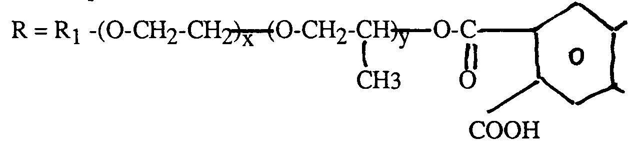 ou 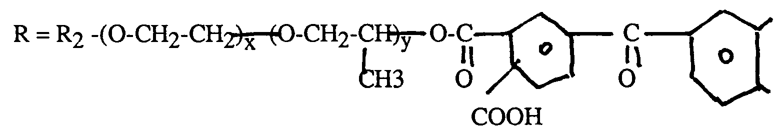 avec
x et y sont des nombres allant de 0 à 100 inclus tels que la somme (x + y) varie de 0 à 100 inclus
R₁ est un radical alkyle pouvant contenir jusqu'à 40 atomes de carbone si x ou y ne sont pas nuls et possédant de 10 à 40 atomes de carbone si x et y sont nuls, ou bien un radical aryle, alkylaryle, arylalkyle, polyaryle ramifié ou non, pouvant chacun comporter un ou plusieurs groupements fonctionnels du type carboxyle, ester, éther, nitro, amine tertiaire, amide, imide, nitrile, halogène ou uréthanne
R₂ est un radical alkyle pouvant contenir jusqu'à 40 atomes de carbone quels que soient x et y, ou bien un radical aryle, alkylaryle, arylalkyle, polyaryle ramifié ou non, pouvant chacun comporter un ou plusieurs groupements fonctionnels du type carboxyle, ester, éther, nitro, amine tertiaire, amide, imide, nitrile, halogène ou uréthanne.

**8/** Utilisation d'un agent dispersant selon la revendication 7 à l'obtention de composition polymère chargée selon l'une des revendications 1 à 6.

**9/** Procédé de fabrication des compositions polymères chargées, fluides et homogènes selon l'une quelconque des revendications 1 à 6 caractérisé en ce que l'agent dispersant peut-être ajouté sur la charge minérale et/ou organique avant l'introduction dans la résine soit dans la résine avant l'introduction de la charge minérale et/ou organique soit dans la résine après l'introduction de la charge minérale et/ou organique.

**10/** Application de la composition polymère chargée, fluide et homogène selon l'une des revendications 1 à 6 à la fabrication des matières plastiques.
